# EUROPEAN PATENT APPLICATION

(11) **EP 4 488 340 A1**
(43) Date of publication of application: **08.01.2025**
(21) Application number: 23763693.1
(22) Date of filing: 28.02.2023
(51) Int. Cl.: C09D 7/61, C09D 11/037, C09D 129/04, C08K 3/22, C08K 3/34, C08K 3/26

(54) **COLORANT COMPOSITION FOR FILM COATING SUITABLE FOR UV LASER PRINTING AND UV LASER PRINTING METHOD USING SAME COMPOSITION**

(30) Priority: 02.03.2022 KR 20220026538
(71) Applicant: ROQUETTE FRERES, 62136 Lestrem (FR)
(72) Inventor: LEE, Kyoung-Ho, Suwon-si, Gyeonggi-do 16328 (KR)
(74) Representative: Engelhard, Markus
(86) International application number: PCT/KR2023/002821
(87) International publication number: WO 2023/167494

(57) **Abstract**

An aspect of the present invention provides a composition for film coating, which can replace titanium oxide suspected of being carcinogenic and, especially, a composition for film coating suitable for laser printing after film coating. Another aspect of the present invention provides a composition for film coating, which has an excellent white coloring effect and a reduction in scuffing. Another aspect of the present invention provides a method for laser printing on tablets or capsules by using the composition according to the present invention.

## Description

### [Technical Field]

This application claims priority based on Korean Patent Application No. 10-2022-0026538 filed on March 2, 2022, and all contents disclosed in the specification and drawings of that application are incorporated herein by reference.

The present disclosure relates to colorant(s) that can replace titanium oxide, a colorant suspected of being carcinogenic, and a film coating composition comprising such colorant(s). In particular, the present disclosure relates to a film coating composition suitable for laser printing. The present invention also relates to a method for coating a tablet or capsule using such a film coating composition and performing UV laser printing thereon.

### [Background Art]

Titanium oxide (TiO₂) has been commonly used as a white colorant in polymer film coating of tablets or capsules.

In addition, it is common to laser print a specific mark on the surface of tablets or capsules for identification, and titanium dioxide has been recognized as an essential component that enables such laser printing.

However, the potential carcinogenicity of titanium oxide has become an issue, as the European Commission recently classified titanium dioxide (TiO₂) as a carcinogen. Therefore, the use of titanium oxide is being discontinued, and an alternative colorant that can express white or hide other colors of the tablet is needed. Meanwhile, a UV laser printer that is commonly used enables printing by inducing color development of titanium by removing oxygen from titanium oxide. Therefore, it is currently impossible to print using a UV laser printer without using titanium oxide. Thus, a film coating composition that enables laser printing without containing titanium dioxide is urgently needed. That is, there is a pressing need for a method that can coat tablets or capsules without using titanium dioxide, and furthermore, enable laser printing thereon.

### [Disclosure]

### [Technical Problem]

Therefore, the problem that the present invention seeks to solve is to provide a new colorant that can replace the existing colorant, titanium oxide, and a film coating composition comprising the same.

In particular, the present invention seeks to provide a film coating composition that can be laser printed.

Furthermore, the present invention provides a method of film coating a tablet or capsule using the film coating composition and laser printing thereon.

### [Technical Solution]

In order to achieve the above technical object, an embodiment of the present disclosure provides a film coating polymer composition for film coating of a tablet or capsule, comprising (a) zinc oxide and (b) at least one selected from the group consisting of talc, calcium carbonate, and magnesium oxide. Preferably, the film coating polymer composition according to the present disclosure comprises (a) zinc oxide and (b) talc.

Preferably, in the present invention, the tablet or capsule is laser-printed after film coating, and the film coating formed with the film coating composition according to the present disclosure is suitable for such laser printing.

In a preferred embodiment of the present disclosure, the content of (b) talc, calcium carbonate, magnesium oxide, or a mixture thereof in the composition is 5-40 wt% (preferably 5-35 wt%) based on the total weight of the remaining dry materials excluding the coating solvent, and the content of (a) zinc oxide is 1-30 wt% (preferably 1-20 wt%, more preferably 1-10 wt%) based on the total weight of the remaining dry materials excluding the coating solvent.

In the present invention, unless otherwise stated, the content is based on the total weight of the remaining dry materials excluding the coating solvent.

In a preferred embodiment of the present invention, the film coating composition according to the present invention further comprises a plasticizer, and the plasticizer may be triacetin, polyethylene glycol, lecithin, or a mixture thereof. For the purpose of the present invention, which is laser printing, the film coating composition according to the present disclosure preferably comprises polyethylene glycol as the plasticizer. Such polyethylene glycol may include polyethylene glycol 400, polyethylene glycol 3350, polyethylene glycol 4000, polyethylene glycol 6000, etc., and for the purpose of the present invention, the polyethylene glycol series does not affect printing.

Such plasticizer may be used in an amount of 3-25 wt% relative to the total weight of the film coating composition, and 10-20 wt% is preferred.

According to the present invention, in the film coating composition for forming a film layer suitable for laser printing, polyvinyl alcohol, hypromellose, polyvinylpyrrolidone, methacrylic acid copolymer, hydroxypropyl cellulose, or a mixture thereof may be used as a polymer for forming a film layer. Such a film coating polymer may be used in an amount of 30-75 wt% relative to the total weight of the film coating composition, preferably 40-70 wt%, and more preferably 40-60 wt%.

The film coating composition of the present invention may optionally additionally comprise a colored pigment in addition to the white colorants. Such a colorant may be a pigment of the aluminum lake series, an iron oxide pigment, a natural pigment such as gardenia, etc., which may be used alone or in combination, and may be contained in an amount of 0.01-8 wt% relative to the total weight of the film coating composition within a range that does not inhibit the content ratios mentioned above.

The film coating composition of the present invention may be provided in the form of a solid powder, and may also be provided in the form of a coating solution by mixing with an appropriate solvent. When the composition of the present invention is provided as a coating solution, the content of each component is evaluated based on the total weight of the remaining dry materials excluding the coating solvent.

The coating solvent may preferably be selected from the group consisting of water, ethanol, and mixtures thereof.

The present invention also provides a method for UV laser printing of a tablet or a capsule, which comprises the steps of (S1) film-coating the tablet or capsule using the film coating composition according to the present invention and (S2) printing the film-coated tablet or capsule using a laser, preferably a UV laser.

The laser device and laser light for printing (marking) on the coated film using a UV laser are not particularly limited, and the laser medium may be emitted in any one of a solid state, a liquid state, and a gaseous state. As the solid laser, a YLF laser, a YAG laser, a YVO4 laser, a fiber laser, etc. may be used, but are not limited thereto.

### [Advantageous Effects]

One aspect of the present invention provides a film coating composition capable of forming a film layer suitable for laser printing without using titanium oxide, which is suspected of being carcinogenic. Tablets or capsules coated with the film coating composition of the present invention have an excellent white coloring effect, reduced scuffing phenomenon, and are suitable for laser printing.

### [Brief Description of Drawings]

Figure 1 shows the results of photographs taken after laser printing on white tablets film-coated with one example composition according to the present invention.
Figure 2 shows the results of photographs taken after laser printing on tablets film-coated with comparative example compositions.
Figure 3 shows the results of photographs taken after laser printing on colored tablets film-coated with one example composition according to the present invention.

### [Mode for Invention]

Hereinafter, examples and the like will be described in detail to help understanding of the present invention. However, the examples according to the present invention may be modified in various different forms, and the scope of the present invention should not be construed as being limited to the following examples. The examples of the present invention are provided to more completely explain the present invention to a person having average knowledge in the field to which the present invention pertains.

### Preparation of white film-coated tablets for UV laser printing

Various film-coated tablets for testing UV laser printing were manufactured. White film-coating was performed on the same tablets with the same formulation as in Table 1 below. Specifically, 60 parts by weight of the coating composition in Table 1 below and about 270 parts by weight of purified water were mixed to prepare coating solutions. Thereafter, film-coating was performed under conditions such as an inlet temperature of 58 °C, a coating pan rotation speed of 30 rpm, a pump speed of 3.5 rpm, and an airflow of 140 m³/hr until the weight of the uncoated tablet (716 mg) increased by 8 wt%. The total coating time was approximately 56 minutes. The coating device used was XENA-IV_{BTC}.

**[Table 1]**

| Ingredient | Exampl e 1 | Exampl e 2 | Exampl e 3 | Exampl e 4 | Exampl e 5 | Exampl e 6 | Exampl e 7 |
|---|---|---|---|---|---|---|---|
| Hypromellose 6 mPa·s | 62.5 | 62.5 | 62.5 | 62.5 | 62.5 | 62.5 | 62.5 |
| Titanium oxide | 31.25 | 0 | 0 | 0 | 0 | 0 | |
| Zinc oxide | 0 | 0 | 25 | 5 | 1 | 0 | 0 |
| Calcium carbonate | 0 | 0 | 0 | 0 | 0 | 31.25 | 0 |
| Magnesium oxide | 0 | 0 | 0 | 0 | 0 | 0 | 31.25 |
| Talc | 0 | 31.25 | 6.25 | 26.25 | 30.25 | 0 | 0 |
| Polyethylene glycol 400 | 6.25 | 6.25 | 6.25 | 6.25 | 6.25 | 6.25 | 6.25 |
| Total | 100 | 100 | 100 | 100 | 100 | 100 | 100 |

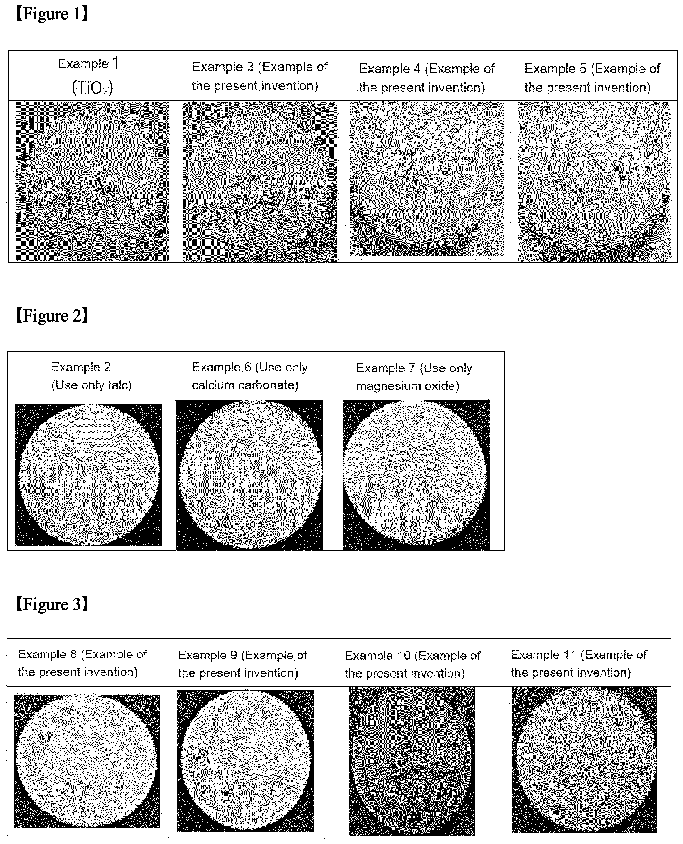

Afterwards, printing tests were performed on the film coating of each tablet using a UV laser printing device (Enclony PLPI, Enclony). The printability was evaluated, and the results are shown in Table 2 below. In addition, photographs of successfully printable film tablets are shown in figure 1, and the results of photographs of examples in which printing was practically impossible are shown in figure 2.

**[Table 2]**

| | Example 1 | Example 2 | Example 3 | Example 4 | Example 5 | Example 6 | Example 7 |
|---|---|---|---|---|---|---|---|
| Printability | Possible | Impossib le | Possible | Possible | Possible | Impossib le | Impossib le |

As shown in Table 2 above, the film coating comprising the titanium oxide was suitable for laser printing. However, in the case of the film coating comprising only talc, calcium carbonate or magnesium oxide, the printing mark was not printed to the extent that it was impossible to identify with the naked eye, so UV laser printing was practically impossible. In contrast, when a small amount of zinc oxide was comprised, laser printing was possible.

### Preparation of colored film-coated tablets for UV laser printing

The effect of the composition of the present invention was evaluated in colored coatings rather than white coatings. Film-coated tablets were manufactured in the same manner as in Example 1 with the formulation of Table 3 below.

**[Table 3]**

| Ingredient | Example 8 | Example 9 | Example 10 | Example 11 |
|---|---|---|---|---|
| Polyvinyl alcohol | 40.0 | 40.0 | 40.0 | 40.0 |
| Zinc oxide | 1.0 | 1.0 | 1.0 | 1.0 |
| Calcium carbonate | 0 | 37.8 | 0 | 0 |
| Talc | 37.8 | 0 | 37.8 | 37.8 |
| Polyethylene glycol 3350 | 20.2 | 20.2 | 20.2 | 20.2 |
| Pigment | 1.0¹⁾ | 1.0²⁾ | 1.0³) | 1.0⁴) |
| Total | 100 | 100 | 100 | 100 |

| | | | | |
|---|---|---|---|---|
| 1) Quinoline Yellow WS Aluminum Lake 2) Sunset Yellow FCF Aluminum Lake 3) Allura Red AC Aluminum Lake 4) Brilliant Blue FCF Aluminum Lake | | | | |

Thereafter, laser printing was performed in the same manner as in the previous experiment, and the results are shown in figure 3. As shown in figure 3, the film coating composition according to the present invention was suitable for laser printing, and the film coating process and the film coating itself produced thereafter were also good.

## Claims

1. A film coating composition **characterized in that** it comprises (a) zinc oxide and (b) at least one selected from the group consisting of talc, calcium carbonate, and magnesium oxide in a polymer composition for film coating of a tablet or capsule.

2. The film coating composition of claim 1, comprising (a) zinc oxide and (b) talc.

3. The film coating composition of claim 1 or 2, wherein the tablet or capsule is laser-printed after the film coating.

4. The film coating composition of claim 1 or 2, wherein the content of (b) talc, calcium carbonate, magnesium oxide, or a mixture thereof in the composition is 5-40 wt% based on the total weight of the remaining dry materials excluding the coating solvent, and the content of (a) zinc oxide is 1-30 wt% based on the total weight of the remaining dry materials excluding the coating solvent.

5. The film coating composition of claim 1, wherein the polymer is polyvinyl alcohol, hypromellose, polyvinylpyrrolidone, methacrylic acid copolymer, hydroxypropyl cellulose, or a mixture thereof.

6. A method for laser printing a tablet or capsule, comprising
(S1) a step of film-coating a tablet or capsule using the film coating composition according to any one of claims 1 to 5; and
(S2) a step of laser printing on the tablet or capsule coated in step S1,

7. The method for laser printing a tablet or capsule of claim 6, wherein the laser is a UV laser.
